# EUROPEAN PATENT APPLICATION

(11) **EP 1 481 655 A1**
(43) Date of publication of application: **01.12.2004**
(21) Application number: 04076574.5
(22) Date of filing: 27.05.2004
(51) Int. Cl.: A61F 13/14

(54) **Support element for supporting a prolapse and support set provided with such a support element**

(30) Priority: 28.05.2003 NL 1023553
(71) Applicant: Groeneveld, Jacqueline, 5263 NW Vught (NL)
(72) Inventor: Groeneveld, Jacqueline, 5263 NW Vught (NL)

(57) **Abstract**

The invention relates to a support set which may be used by a woman suffering from a prolapse. The set comprises at least underpants (1) and support means (2,3) to be worn over the underpants (1), consisting of a hip-belt (2) to which a support belt (3) is connected, made of an elastic material and operationally passing underneath the crotch of the woman.

## Description

The invention relates to a support element, to be used by a woman suffering from a prolapse in the abdomen. A prolapse is a disorder in which muscles and/or connective tissue of a pelvic area of a woman offer insufficient support to organs situated in the pelvic cavity. It is a non life-threatening disorder, but it can seriously reduce the quality of life of the woman. Classical treatments involve physiotherapy, for training the muscles in the pelvic area, the insertion of a diaphragm or an operation, during which the connective tissue is usually shortened in such a way that prolapsed organs are more or less put back to their original positions.

The support element according to the invention renders assistance to women by suppressing the prolapse from the outside, more in particular by applying a force from the outside which at least substantially compensates gravitational forces. The inventive support element is thereto characterised in that the support element operationally passes underneath the crotch of the woman and near the hip area is provided with fixing means for keeping the support element in place. Thereby, the support element delivers an upwards directed force acting onto the crotch of the woman.

A favourable embodiment of the inventive support element is characterised in that the fixing means are provided with a hip-belt, while the fixing means are operationally connected to a front side and a back side of the hip-belt. A support element constructed in this way provide for sufficient support, is relatively comfortable and it can be worn underneath the outer clothes substantially without showing through.

A further favourable embodiment is characterised in that the support element is made of a stretchy material, to the extent that the support belt itself generates the supporting force, while it easily yields to movements of the wearer without the fixing means getting displaced.

A further favourable embodiment is characterised in that support element is provided with a woven-in crotch. A crotch of this type distributes the exerted forces very well and is moreover capable of absorbing some body liquids, which is of great importance, as the occurrence of a prolapse is often accompanied by a minor form of incontinence.

A favourable embodiment according to a further aspect of the invention is characterised in that the support element also comprises a support object, which can be attached to the woven-in crotch. With the aid of this support object, the pressure can be concentrated and it can be used, if desired, to realise a higher pressure at a previously determined location.

A further favourable embodiment is characterised in that the woven-in crotch is made up of two layers, between which the support object may be positioned. In this case, the support object preferably comprises an at least substantially plate-shaped element, made of a flexible material, for example silicon rubber.

A further favourable embodiment is characterised in that the plate-shaped element is provided with at least one cavity, more in particular situated near one or more body openings. Said at least one cavity may, dependent upon the particular need of the woman, be filled with for example silicon oil, which causes at that location a bulge in the plate-shaped element that presses against the body opening. In this way, incontinence may in a number of cases be reduced or even cured.

The invention moreover comprises a support set provided with a support element according to one of the previous claims, as well as underpants to be worn underneath the support element, preferably made of a hydrophillic, slightly stretchy material, free of seams and/or stitching in the crotch area.

The invention also relates to underpants, a support element, hip-belt, support belt or support object as part of such a support set.

The invention will now be further explained with a reference to the following figures, in which:
- Fig. 1: represents a possible embodiment of a support set according to the invention;
- Fig. 2A: represents a possible embodiment of a support belt in top view;
- Fig. 2B: represents this embodiment in side view;
- Fig. 3A: represents a possible embodiment of a support object in top view;
- Fig. 3B: represents this embodiment in side view;
- Fig. 3C: represents this embodiment in side view, with one cavity being filled;
- Fig. 4A: schematically represents a normal position of various organs in the pelvic cavity in cross section;
- Fig. 4B: schematically represents the position of these organs with weakened muscles in the pelvic area, when a support set according to the invention is applied.

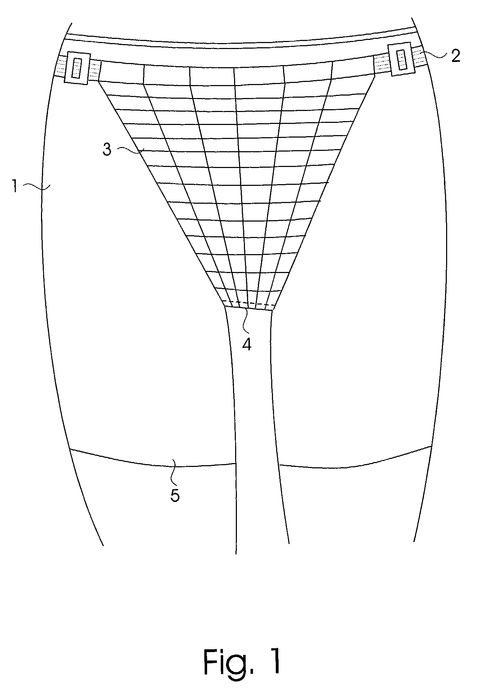

Fig. 1 represents a possible embodiment of a support set according to the invention, here consisting of underpants 1 made of a hydrophillic, slightly stretchy fabric which is to be tolerated well on the skin, for example consisting of cotton and polyester. Over underpants 1, a hip-belt 2 is fitted provided with fastening means like buckles or Velcro, onto which a support belt 3 is attached, made of a strong, elastic material, which passes operationally underneath a crotch and which delivers an upwards directed force onto the crotch by virtue of its elasticity. In the crotch area, support belt 3 is provided with a crotch 4, made of a hydrophillic material, which distributes the exerted force and which is moreover capable of absorbing some body liquids, which is of great importance, as the occurrence of a prolapse is often accompanied by a minor form of incontinence. It is understood that underpants 1, support belt 3 and crotch 4 are made of an easily washable material and that hip-belt 2 must be attached in such a way that it may be easily be removed before washing. Underpants 1 are preferably provided with legs 5, which has the advantage that irritation of the groins by support belt 3 is practically excluded. Legs 5 extend preferably to at least halfway down the upper legs, in order to prevent them from working up. Support belt 3 is for example manufactured of cotton, to which polyester is added for strength and to which for example rubber is added for obtaining elasticity, for example in the proportion of 55% cotton, 28% polyester and 17% rubber.

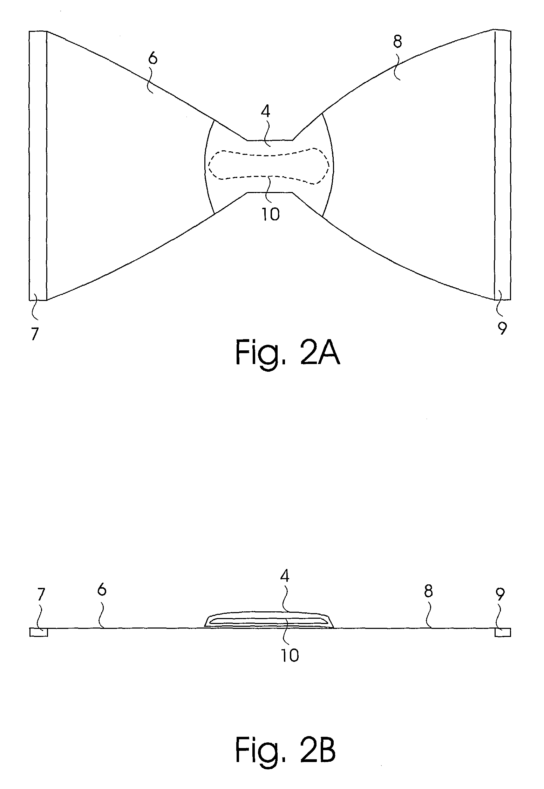

Fig. 2A represents a possible embodiment of a support belt in top view, consisting of a front 6 provided with a turning 7, a back 8 provided with a turning 9 and in the centre a knitted in or attachable crotch 4. Hip-belt 2, shown in Fig. 1, can be pulled through turnings 7,9, with which a simple and sturdy attachment is realised. Crotch 4 consists of two layers, between which a support object 10, made of silicon rubber or a comparable soft material can be placed, with which the pressure exerted to the crotch of a user can be distributed in a previously determined manner.

Fig. 2B represents this embodiment in side view, with front 6, back 8, turnings 7,9, crotch 4 and support object 10.

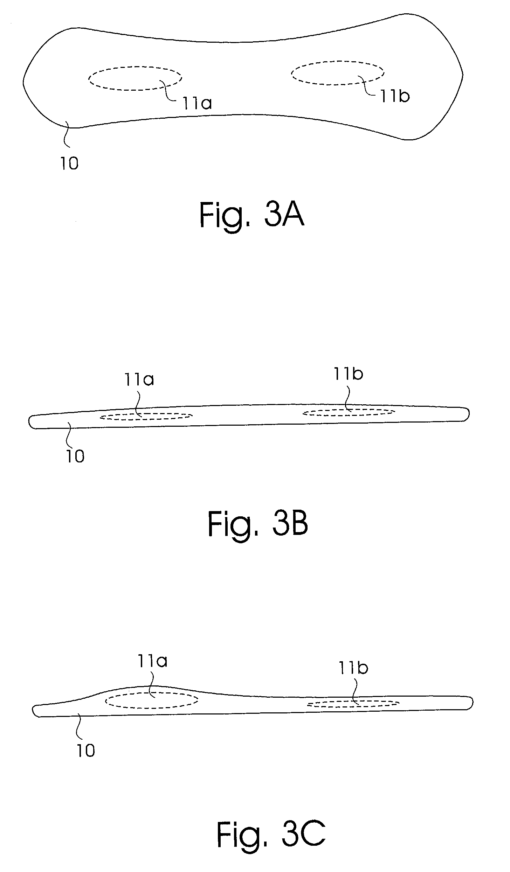

Fig. 3A represents a possible embodiment of a support object 10 in top view, consisting of a plate-shaped part made of silicon rubber, provided with rounded off edges, in which on previously determined locations cavities 11a,11b have been made which may be filled with for example silicon oil, for obtaining pressure points, dependent upon the individual needs of the user.

Fig. 3B represents this embodiment in side view, in a situation in which the cavities have practically not been filled.

Fig. 3C represents this embodiment in side view, with one cavity 11a being filled to an extent that it operationally presses against the exit of the urethra. It turns out that a pressure point of this type significantly reduces the risk of unintentional loss of urine.

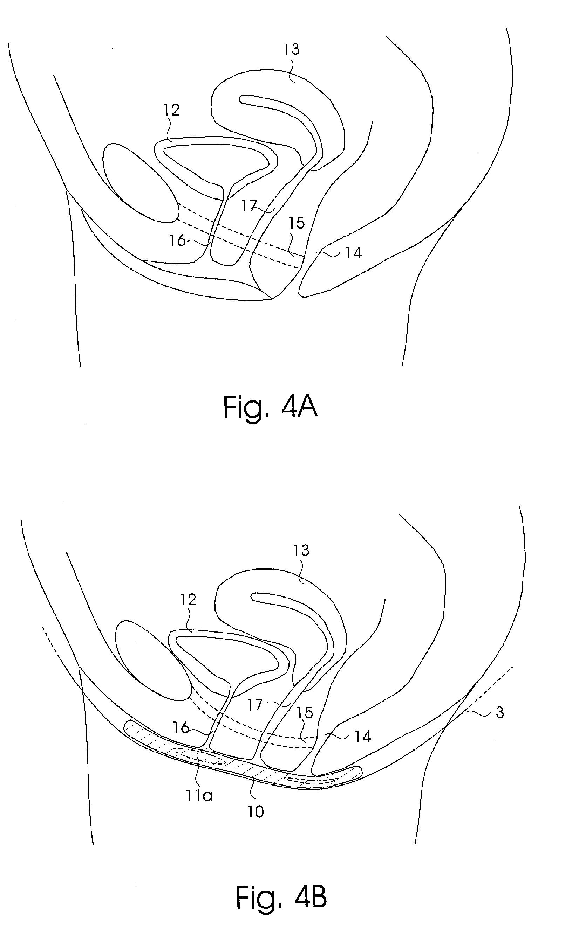

Fig. 4A schematically represents a normal position of various organs in the abdomen in cross section and more in particular of the bladder 12, the uterus 13 and the rectum 14 in the pelvic cavity, which organs are mainly kept in place by muscles 15 making up the pelvic floor, indicated by a dotted line. The pelvic muscles 15 include also the orbicular muscles which close the urethra 16, the vagina 17 and the rectum 14. A prolapse, a situation in which the pelvic muscle tissue and/or the surrounding pelvic connective tissue is seriously weakened, often brings incontinence as a complication, as a result of which the patient may involuntary lose urine and/or faces.

Fig. 4B schematically represents the position of the bladder 12, the uterus 13 and the rectum 14 in the pelvic cavity with weakened muscles 15 making up the pelvic floor, when a support set according to the invention is applied. For clarity reasons, crotch 4 is left out in the figure. Said organs are indeed still slightly prolapsed, but support object 10, which is pulled upwards by support belt 3, precludes a further prolapse. In the embodiment shown here, support object 10 is provided with one filled cavity 11a, which presses against the exit of the urethra, which further reduces the possibility of urine being lost.

The invention is not limited to the embodiment represented by the drawings and described in the previous chapters but it may be varied within the scope of the invention. It is for example possible to construct the support element in such a way that it is no longer necessary to wear underpants underneath. The support element could even be shaped as a pair of underpants, possibly including pipes. In that case, the underpants and the support element are in fact integrated to a single entity. The support element is preferably woven or knit of yarn and elastic fibres, incorporated in two directions in the knitting or fabric in order to obtain an elasticity in two directions. Thereby, the knitting or fabric must be made such that, when worn, especially in the crotch area of the woman a support force is generated which counteracts the prolapse.

The hip-belt may be integrated in the support element as well and may for example exist of a reinforces belt being part of the top side of the support element. The hip-belt may itself be elastic. Instead of or in addition to a hip-belt, in the hip area the support element may be provided on the inside with silicon parts (bumps, strips or the like) which act as gripping means and possibly an elastic belt to be worn round the body, onto which silicon parts have been attached.

## Claims

1. Support element, to be used by a woman suffering from a prolapse in the abdomen, **characterised in that** the support element operationally passes underneath the crotch of the woman and near the hip area is provided with fixing means for keeping the support element in place.

2. Support element according to claim 1, **characterised in that** the fixing means are provided with a hip-belt, while the fixing means are operationally connected to a front side and a back side of the hip-belt.

3. Support element according to claim 1 or 2, **characterised in that** the support element is made of a stretchy material.

4. Support element according to claim 3, **characterised in that** the support element is provided with a woven-in crotch.

5. Support element according to claim 4, **characterised in that** the set also comprises a support object, which can be attached in the woven-in crotch.

6. Support element according to claim 5, **characterised in that** the woven-in crotch is made up of two layers, between which the support object may be positioned.

7. Support element according to claim 5 or 6, **characterised in that** the support object comprises an at least substantially plate-shaped element, made of a flexible material.

8. Support element according to claim 7, **characterised in that** the plate-shaped element is provided with at least one cavity.

9. Support set provided with a support element according to one of the previous claims, as well as underpants to be worn underneath the support element, preferably made of a hydrophillic, slightly stretchy material, free of seams and/or stitching in the crotch area.

10. Underpants, support element, a hip-belt, support belt or support object as part of a support set according to claim 9.
